# EUROPEAN PATENT APPLICATION

(11) **EP 3 251 622 A1**
(43) Date of publication of application: **06.12.2017**
(21) Application number: 17173893.3
(22) Date of filing: 01.06.2017
(51) Int. Cl.: A61B 18/14, A61B 18/00, A61B 90/00

(54) **BALLOON CATHETER AND RELATED IMPEDANCE-BASED METHODS FOR DETECTING OCCLUSION**

(30) Priority: 02.06.2016 US 201615172118
(71) Applicant: Biosense Webster (Israel) Ltd., Yokneam 2066717 (IL)
(72) Inventor: KEYES, Joseph Thomas, Irwindale, CA 91706 (US); BEECKLER, Christopher Thomas, Irwindale, CA 91706 (US)
(74) Representative: Carpmaels & Ransford LLP

(57) **Abstract**

A system for assessing occlusion of a region to blood flow includes a catheter with an inflatable member, a first electrode configured for placement upstream of the inflatable member and the second electrodes configured for placement downstream of the inflatable member, the inflatable member configured for inflation to occlude the blood flow through the region. The system further includes a current/voltage source, a resistor and a voltmeter, wherein these components along with the first and second electrodes are configured to form an impedance measuring circuit configured to detect a change in impedance for indicating occlusion of the region to the blood flow.

## Description

### FIELD OF INVENTION

This invention relates to electrophysiologic (EP) catheters, in particular, EP catheters for mapping and/or ablation in the heart.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Important sources of undesired signals are located in various tissue regions in or near the heart, for example, the atria and/or and adjacent structures such as areas of the pulmonary veins, and left and right atrial appendages. Regardless of the sources, unwanted signals are conducted abnormally through heart tissue where they can initiate and/or maintain arrhythmia.

Procedures for treating arrhythmia include surgically disrupting the origin of the signals causing the arrhythmia, as well as disrupting the conducting pathways for such signals. More recently, it has been found that by mapping the electrical properties of the heart muscle in conjunction with the heart anatomy, and selectively ablating cardiac tissue by application of energy, it is possible to cease or modify the propagation of unwanted electrical signals from one portion of the heart to another. The ablation process destroys the unwanted electrical pathways by formation of non-conducting lesions.

A typical ablation procedure involves the insertion of a catheter having electrode(s) at its distal end into a heart chamber. An indifferent electrode is provided, generally adhered to the patient's skin. Radio frequency (RF) current is applied to the electrode(s), and flows between the surrounding media, i.e., blood and tissue and the indifferent electrode. The distribution of current depends on the amount of electrode surface in contact with the tissue, as compared to blood which has a higher conductivity than the tissue. Heating of the tissue occurs due to Joule heating. If the tissue is heated sufficiently, protein denaturation occurs; this in turn forms a lesion within the heart muscle which is electrically non-conductive.

A focal catheter works well, for example, when ablating a line of block in the atria. However, for tubular regions in or around the heart, this type of catheter is cumbersome, skill dependent, and time consuming. For example, when the line of block is to be made about a circumference of the tubular region, it is difficult to manipulate and control the distal end of a focal catheter so that it effectively ablates about the circumference. In current practice a line of block is accomplished by maneuvering the catheter from point to point and is highly dependent on the skill of the operator and can suffer from incomplete isolation of target areas such as the pulmonary vein ostia. However, done well, it can be very effective.

Catheters with circular ablation assemblies (or "lasso-type" catheters) are known. This type of catheter comprises a catheter body having at its distal end an ablation assembly with a preformed generally circular curve with an outer surface and being generally transverse to the axis of the catheter body. In this arrangement, the catheter has at least a portion of the outer circumference of the generally circular curve in contact with the inner circumference or ostium of a tubular region in or near the patient's heart, e.g., a pulmonary vein.

Ablation catheters with inflatable assemblies or balloons are also known. Such balloons may include electrodes positioned on the outer surface of the balloons for ablating tissue and are typically inflated with a pressurized fluid source. More recently, inflatable catheter electrode assemblies have been constructed with flex circuits to provide the outer surface of the inflatable electrode assemblies with a multitude of very small electrodes. Examples of catheter balloon structures are described in U.S. Application Serial No. 14/578,807, entitled Balloon for Ablation Around Pulmonary Vein, the entire content of which is incorporated herein by reference.

Atrial fibrillation (AF) is an abnormal heart rhythm that originates in the atria of the heart. The occurrence of AF appears to be dependent on a number of factors, including the presence of intra-atrial conduction delay. A common initiating factor for AF appears to be atrial ectopic beats, where the major sources of these appear to be the pulmonary veins. Accordingly, an ablation procedure commonly referred to as Pulmonary Vein Isolation (PVI) seeks to electrically isolate the pulmonary veins from the left atrium by a circumferential line of ablation at or near the ostia as the junctions between the pulmonary veins and the left atrium.

Ablation catheters with inflatable assemblies or balloons have been used for PVI. The balloons include electrodes on its outer surface that are adapted for making circumferential contact with tissue lining a tubular region of the pulmonary vein. To assess and ensure a balloon and its electrodes are in optimum circumferential contact with the inner tissue wall of a pulmonary vein, occlusion of the pulmonary vein can be verified by, for example, ultrasound or fluoroscopy. With fluoroscopy, X-ray and contrast dye enable visualization of blood flow from the pulmonary vein into the left atrium, and hence occlusion of the pulmonary vein. However, carried by blood flow, the contrast dye can dissipate quickly, and prolonged exposure to X-ray and increased contrast dye loading may pose health risks to the patients.

Accordingly, a need exists for a catheter with a balloon that can detect complete occlusion of an ostium or a tubular region for assessing tissue contact, and correspondingly, ensure that an ostium or a tubular region is not fully occluded. It is desirable that such a balloon catheter utilizes existing electrical pathways of the geometry of the pulmonary veins and repurposes existing features on conventional balloon catheters to provide added functionality to ring electrodes in addition to electrograms or location sensing capabilities, in devising a low-cost, easy-to-implement solution that can implemented on balloon ablation equipment to determine proper contact. Such a balloon catheter may carry electrodes on the surface of the balloon for circumferential sensing and/or ablation capabilities.

### SUMMARY OF THE INVENTION

Electrical impedance is a measurement of how electricity travels through tissue. Every tissue, including soft tissue, hard tissue and bodily fluids, such as blood, has different electrical impedance determined by its molecular composition. Because certain tissue conducts electrically better than other tissue, certain tissue has lower electrical impedance while other tissue has higher electrical impedance. The present invention advantageously utilizes this difference, and recognizes that by placing two electrodes to encounter and contact blood flow through a region in defining a conduction pathway in the blood flow between the two electrodes, and passing a low level electrical current between the two electrodes along the conduction pathway, a change in the measured impedance of the conduction pathway between the two electrodes can provide an indication of a change in the blood flow, including occlusion of the region to the blood flow.

In some embodiments of the present invention, a method of detecting a change in tissue includes providing an upstream electrode and a downstream electrode in relation to blood flow through a region, passing an electrical current between the upstream and downstream electrodes, occluding the region, and monitoring a change in impedance of the electrical current between the electrodes to assess occlusion in the region to the blood flow. In some more detailed embodiments of the present invention, a method of detecting a change in tissue includes providing an upstream electrode and a downstream electrode in relation to blood flow through a region, inflating a balloon member to occlude the region, passing an electrical current between the upstream and downstream electrodes, and monitoring a change in impedance of the electrical current between the electrodes to assess occlusion in the region to blood flow. In some more detailed embodiments, the monitoring a change in impedance includes monitoring a rate of change of impedance.

In some embodiments of the present invention, a catheter includes an inflatable member, a first electrode at one side of the inflatable member and a second electrode at a generally opposite side of the inflatable member, one of the first and second electrodes configured for placement upstream of blood flow and the other of the first and second electrodes configured for placement downstream of the blood flow, the inflatable member configured for inflation to form an occlusion in the region, the first and second electrodes configured to define a conduction pathway through the region, wherein a change in a measured impedance of the conduction pathway between the electrodes is indicative of the occlusion of the region to the blood flow.

In some embodiments of the present invention, a system for assessing occlusion of a region to blood flow, the system includes a catheter with an inflatable member, a first electrode at a first location relative to the inflatable member and the second electrode at a second location relative to the inflatable member, one of the first and second electrodes configured for placement upstream of the blood flow and the other of the first and second electrodes configured for placement downstream of the blood flow, the inflatable member configured for inflation to occlude the blood flow through the region. The system further includes a current/voltage source, a resistor and a voltmeter, wherein the first and second electrodes are configured to form an impedance measuring circuit with the current/voltage source, the resistor and the voltmeter, and wherein the circuit is configured to detect a change in impedance for indicating occlusion of the region to the blood flow. In more detailed embodiments, the system includes a controller with a processor configured to monitor a rate of change of impedance for indicating occlusion of the region to the blood flow.

In more detailed embodiments, an electrophysiology catheter includes an inflatable member configured for placement in blood flow through a region, the member configured to adopt an inflated configuration in the region, a first electrode distal of the inflatable member and configured for placement upstream of the blood flow, a second electrode proximal of the inflatable member and configured for placement downstream of the blood flow, the first and second electrodes configured to define a conduction pathway, and a first lead wire connected to the first electrode and a second lead wire connected to the second electrode, the first and second lead wires configured to pass an electrical current between the first and second electrodes for measuring impedance of the conduction pathway.

In more detailed embodiments, the catheter includes a shaft extending through the inflatable member, wherein the first electrode is mounted on the shaft distal of the inflatable member and the second electrode is mounted on the shaft proximal of the inflatable member.

In more detailed embodiments, the inflatable member in the inflated configuration is configured to fully occlude the region from blood flow.

In more detailed embodiments, the inflatable member has an outer surface and a plurality of ablation electrodes are affixed to the outer surface, the plurality of ablation electrodes configured for circumferential contact with tissue wall of the region when the inflatable member is in the inflated configuration.

In other embodiments, an electrophysiology system for detecting occlusion, includes the foregoing catheter, an impedance measuring unit electrically connected to the first and second lead wires, the unit configured to provide an output signal representative of a measured impedance of the conduction pathway, an impedance controller configured to receive the output signal, and an alarm responsive to the impedance controller configured to provide an indication to a user.

In more detailed embodiments, the impedance measuring unit includes a current/voltage source, a resistor, and a voltmeter.

In more detailed embodiments, the impedance controller is configured to determine an occurrence of a measured impedance exceeding a threshold impedance value.

In more detailed embodiments, the impedance controller is configured to determine a rate of change of measured impedance.

In more detailed embodiments, the impedance controller is configured to determine an occurrence of a rate of change of measured impedance exceeding a threshold rate of change of impedance.

In more detailed embodiments, the impedance controller includes an A/D converter, an impedance processor, a memory and a clock.

In other embodiments, an electrophysiology catheter for use in or near a tubular region of a patient's heart, comprising an inflatable member configured for placement in blood flow through the tubular region, the inflatable member configured to adopt an inflated configuration in or near the tubular region, a first electrode distal of the inflatable member and configured for placement upstream of the blood flow, a second electrode proximal of the inflatable member and configured for placement downstream of the blood flow, the first and second electrodes configured to define a conduction pathway, and a first lead wire connected to the first electrode and a second lead wire connected to the second electrode, the first and second lead wires configured to pass an electrical current between the first and second electrodes for measuring impedance of the conduction pathway.

In other embodiments, an electrophysiology system for detecting occlusion, comprises a catheter having an inflatable member configured for placement in blood flow from a pulmonary vein into a left atrium through an ostium, the inflatable member configured to adopt an inflated configuration in or near the ostium, a first electrode distal of the inflatable member and configured for placement in the pulmonary vein, a second electrode proximal of the inflatable member and configured for placement in the left atrium, the first and second electrodes configured to define a conduction pathway between the pulmonary vein and the left atrium, and a first lead wire connected to the first electrode and a second lead wire connected to the second electrode, the first and second lead wires configured to pass an electrical current between the first and second electrodes for measuring impedance of the conduction pathway. The system also includes an impedance measuring unit electrically connected to the first and second lead wires, the unit configured to provide an output signal representative of a measured impedance of the conduction pathway, an impedance controller configured to receive the output signal, and an alarm responsive to the impedance controller configured to provide an indication to a user.

### BRIEF DESCRIPTION OF THE DRAWINGS

These and other features and advantages of the present invention will be better understood by reference to the following detailed description when considered in conjunction with the accompanying drawings wherein:
FIG. 1 is a top plan view of a catheter of the present invention, in accordance with one embodiment.
FIG. 2 is a side, partially cross-sectional, view of an electrode assembly of FIG. 1.
FIG. 3A is a schematic representation of the electrode assembly of FIG. 2 in or near an ostium of a pulmonary vein, the assembly in a collapsed configuration.
FIG. 3B is a schematic representation of the electrode assembly of FIG. 2 in or near an ostium of a pulmonary vein, the assembly in an expanded or inflated configuration.
FIG. 4 is a block diagram of an impedance measuring unit or circuit of the present invention, in accordance with one embodiment.
FIG. 5 is a block diagram of an impedance measuring unit or circuit of the present invention, in accordance with another embodiment.
FIG. 6 is a graph illustrating measured impedance difference between bovine blood, and bovine myocardium.

### DETAILED DESCRIPTION OF THE INVENTION

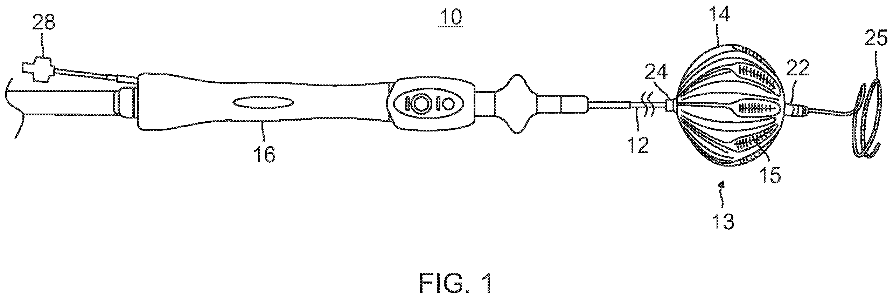

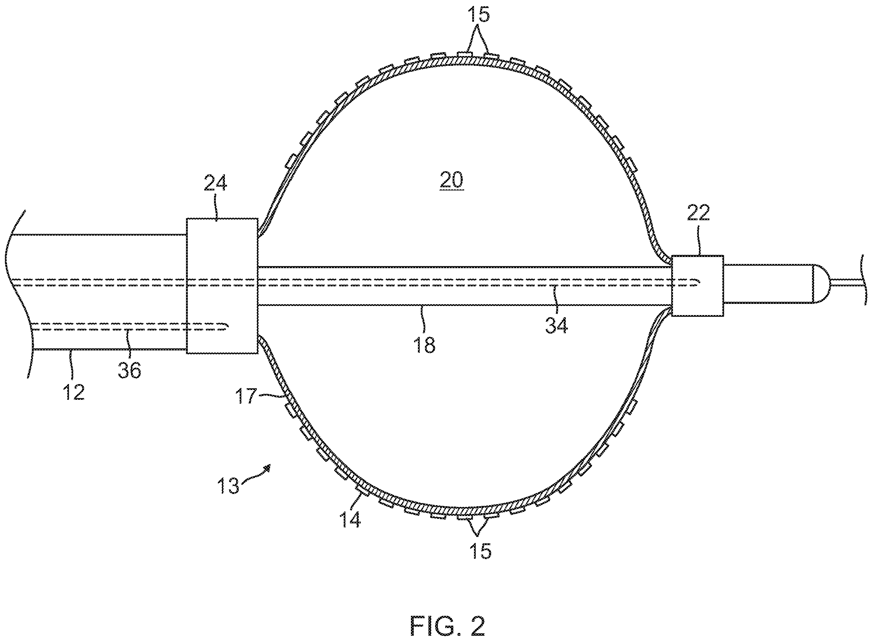

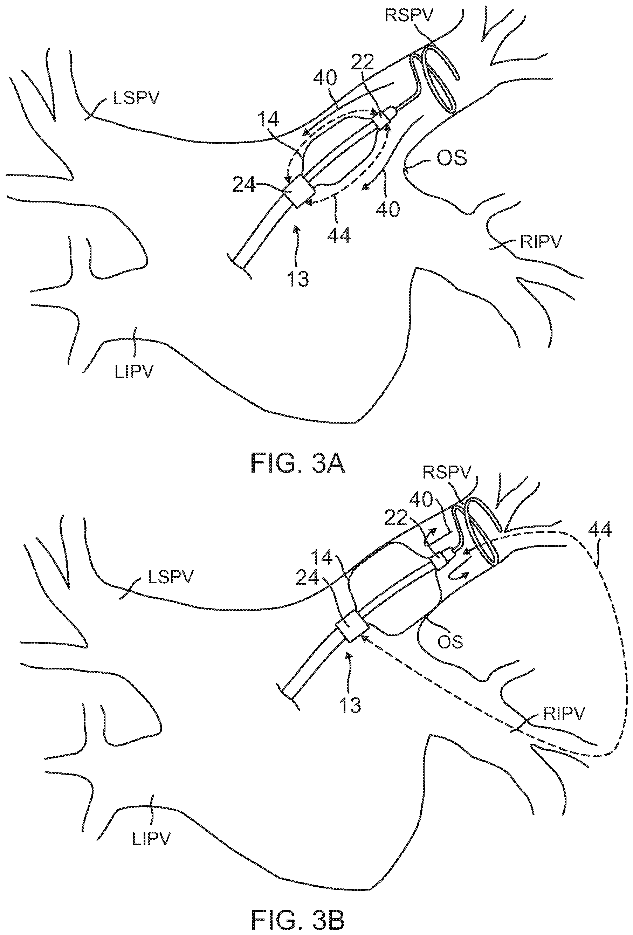

As shown in FIGS. 1 and 2, the catheter 10 comprises an elongated catheter shaft 12, an electrode assembly 13 with an expandable and/or inflatable balloon member 14 having on its outer surface 17 a plurality of electrodes 15, and a support shaft 18 defining a longitudinal axis of the assembly 13 and extending centrally through an interior cavity 20 of the balloon member 14 and carrying distal and proximal ring electrodes 22 and 24 which may be function as radiopaque markers and strengthen attachment of the balloon member to the shaft. However, in embodiments of the present invention, each electrode is rendered "active" by an electrical connection, e.g., a lead wire within the catheter, for receiving and/or transmitting an electrical signal from or to the patient's body and tissues thereof. In the illustrated embodiments, lead wires 34 and 36 are connected to the ring electrodes 22 and 24, respectively. The catheter may also include a distal electrode assembly, for example, a "lasso" assembly 25 having a generally straight proximal portion and a circular distal portion. In some embodiments, the lasso assembly 25 is configured to contact tissue in a tubular region, such as a pulmonary vein RSPV, and the assembly 13 and its balloon member 14 are configured to sit in an ostium OS of the pulmonary vein, as shown in FIG. 3. The catheter 10 includes a control handle 16 attached to the proximal end of the catheter body 12, as shown in FIG. 1.

With reference to FIG. 2, the outer surface 17 of the balloon member 14 carries a plurality of electrodes 15 affixed thereon. The electrodes may be provided by one or more flex circuits affixed to the outer surface of the balloon member, as described in U.S. Application Serial No. 14/578,807, entitled BALLOON FOR ABLATION AROUND PULMONARY VEINS, the entire content of which is incorporated herein by reference. The balloon member 14 assumes a collapsed or deflated (completely or partially) configuration when the catheter is introduced into a patient's vasculature, as shown in FIG. 3A. The balloon member 14 assumes an inflated or expanded configuration when it reaches a target site, such as the ostium OS of the pulmonary vein RSPV, as shown in FIG. 3B. The balloon member 14 is inflated when its interior cavity 18 receives fluid that is supplied by a remote fluid source (not shown) and passed via a luer hub 28 (FIG. 1) into an irrigation tubing (not shown) that extends through the control handle 16, the catheter shaft 12 and into the interior cavity 20 of the balloon member 14.

The balloon member 14 is supported at its proximal and distal ends by the support shaft 18. The distal ring electrode 22 is mounted on the shaft 18 at a location distal of the balloon member 14. The proximal ring electrode 24 is mounted on the shaft 18 at a location proximal of the balloon member 14.

As shown in FIG. 3A, the inflatable electrode assembly 13 with the balloon member 14 in a generally collapsed configuration is inserted into the ostium O of the pulmonary vein PV where blood flow (as shown by arrows 40) continues to flow along a route or initial conduction pathway 44 from the pulmonary vein PV, through the ostium and into the left atrium LA. As such, the distal ring electrode 22 is upstream of the assembly 13 with the balloon member 14 and the proximal ring electrode 24 is downstream of the assembly with the balloon member 14. Because blood has a lower impedance than surrounding cardiac muscle tissue, the current that passes through the lead wire 34 and 36 passes between the distal electrode 22 and to the proximal electrode 24 via the conduction pathway 44 through the blood flow passing through the ostium OS from the pulmonary vein RSPV into the left atrium LA. As the balloon member 14 is inflated, the current is maintained along the conduction pathway 44 so long as blood flow continues to flow from the pulmonary vein RSPV into the left atrium LA, in contact with both the electrodes 22 and 24.

When the balloon member 14 is sufficiently inflated to a desired or appropriate level to fully occlude the ostium OS thus completely occluding blood flow from the pulmonary vein RSPV into the left atrium LA, as shown in FIG. 3B , the current can no longer pass between the electrode 22 and 24 via the ostium OS. The conduction pathway 44 is forced to alter and travel through cardiac muscle tissue or other surrounding tissue, which results in a greater impedance for the conduction pathway.

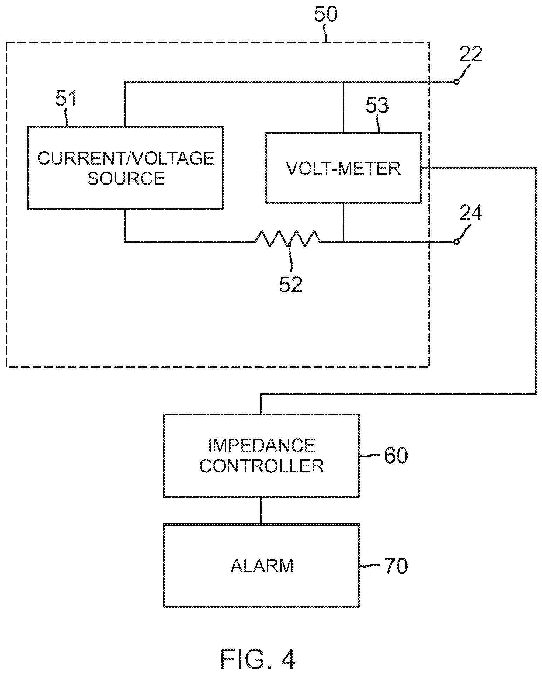

As shown in FIG. 4, some embodiments of the present invention include a circuit for detecting changes in bipolar impedance in the circuit. Embodiments of the present invention recognize that a detection of an increase in impedance greater than a threshold impedance increase indicates complete occlusion and abatement of the blood flow through the ostium OS and thus a change in the conduction pathway from a passage in blood flow through the ostium OS to a passage through surrounding tissue. Such detection advantageously serves as a single reliable indicator of full circumferential contact between the balloon member 14 of the assembly 13 and the ostium OS. With full circumferential contact being an ideal arrangement between the electrodes 15 and tissue wall of the ostium, the electrodes 15 are in prime contact for delivering RF to the ostium in a pulmonary vein isolation procedure. In ablation scenarios, the degree of electrode contact with tissue wall can significantly alter ablation efficacy. With the balloon member in full circumferential contact to ensure full PV occlusion, the pulmonary vein can be completely isolated. It is understood that full circumferential contact is beneficial for a number of procedures beyond RF ablation, including, for example, cryo-ablation, angioplasty, valvulolasty, and pulmonary dilation. Moreover, it is understood that the embodiments of the present invention also have applications where complete occlusion and abatement of blood flow in a region is not desired or is to be avoided.

The electrodes 22 and 24 are rendered active by respective lead wires 34 and 36 that connect the electrodes to one or more circuits, including an impedance measuring unit or circuit 50, as shown in FIG. 4. In some embodiments, the unit or circuit 50 includes a current/voltage source 51, a resistor 52 and a voltmeter 53. The current/voltage source 51, which may, for example, include an alternating current source with a frequency ranging between about 5kHz to 500 kHz, provides an AC current between the electrodes 22 and 24 via the lead wires 34 and 36. The voltage difference between electrodes 22 and 24, combined with the known AC current, for example, approximately 1mA, allows for calculation of the impedance. It is understood by one of ordinary skill in the art that the unit or circuit 50 may be arranged in any suitable configuration, for example, including a voltage source rather than a current source, with any suitable components, where such components may be housed in the catheter handle 16 and/or elsewhere remote from the handle.

In some embodiments of the present invention, as shown in FIG. 4, output of the voltmeter 53 is supplied to an impedance controller 60 which is configured to actuate an alarm 70 in response to the output of the voltmeter 53. The impedance controller 60 is configured to determine whether the output representative of an impedance measurement exceeds a predetermined threshold impedance value, and to trigger the alarm 70 upon such occurrence. The alarm 70 provides the user with an indication of the occurrence of complete occlusion with full abatement of blood flow. The alarm 70 may provide a visual and/or audio signal to the user.

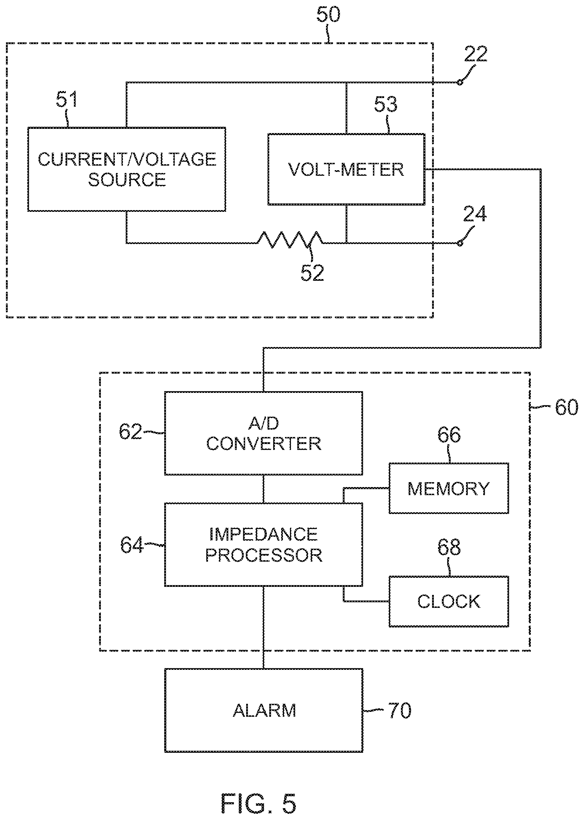

In some embodiments of the present invention, as shown in FIG. 5, the impedance controller 60 is configured to measure a rate of change of the measured impedance. The controller may include an A/D converter 62, an impedance processor 64, a memory 66 and clock 68, wherein the impedance processor 64 samples output from the voltmeter 53 over a predetermined time period to determine whether a rate of change of measured impedance exceeds a predetermined threshold rate of change of impedance, and to trigger the alarm 70 upon such occurrence.

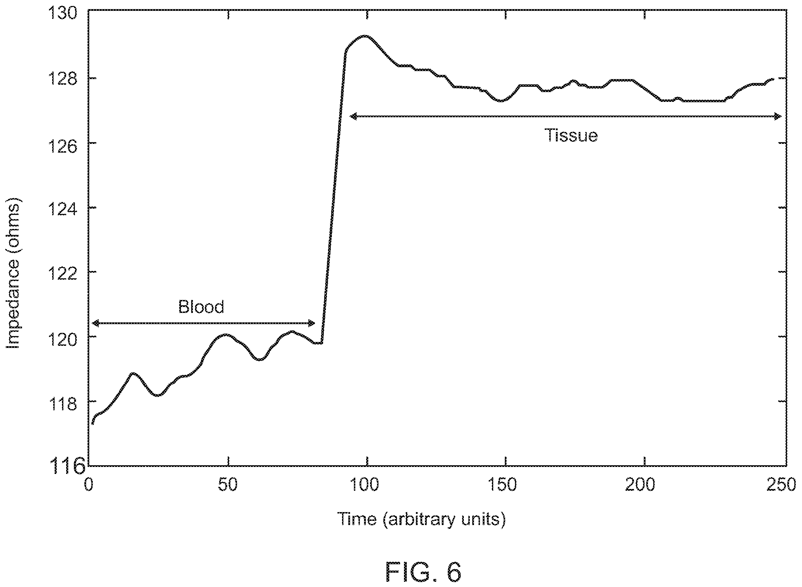

FIG. 6 is a graph illustrating measured impedance difference between bovine blood ranging between about 117 ohms and 120 ohms, and bovine myocardium ranging between about 127 and 129 ohms. Utilizing the rate of change can overcome differences in anatomy and impedance as well as impedance drift during the procedure due to fluid loading.

The preceding description has been presented with reference to presently disclosed embodiments of the invention. Workers skilled in the art and technology to which this invention pertains will appreciate that alterations and changes in the described structure may be practiced without meaningfully departing from the principal, spirit and scope of this invention. As understood by one of ordinary skill in the art, the drawings are not necessarily to scale and any feature or combinations of features described in any one embodiment may be incorporated into any other embodiments or combined with any other feature(s) of other embodiments, as desired or needed. Accordingly, the foregoing description should not be read as pertaining only to the precise structures described and illustrated in the accompanying drawings, but rather should be read consistent with and as support to the following claims which are to have their fullest and fair scope.

## Claims

1. An electrophysiology catheter comprising:
an inflatable member configured for placement in blood flow through a region, the member configured to adopt an inflated configuration in the region;
a first electrode distal of the inflatable member and configured for placement in the blood flow upstream of the inflatable member, a second electrode proximal of the inflatable member and configured for placement in the blood flow downstream of the inflatable member, the first and second electrodes configured to define a conduction pathway; and
a first lead wire connected to the first electrode and a second lead wire connected to the second electrode, the first and second lead wires configured to pass an electrical current between the first and second electrodes for measuring impedance of the conduction pathway.

2. The catheter of claim 1, further comprising a shaft extending through the inflatable member, wherein the first electrode is mounted on the shaft distal of the inflatable member and the second electrode is mounted on the shaft proximal of the inflatable member.

3. The catheter of claim 1, wherein the inflatable member in the inflated configuration is configured to fully occlude the region from blood flow.

4. The catheter of claim 1, wherein the inflatable member has an outer surface and a plurality of ablation electrodes are affixed to the outer surface, the plurality of ablation electrodes configured for circumferential contact with tissue wall of the region when the inflatable member is in the inflated configuration.

5. An electrophysiology system for detecting occlusion, comprising
a catheter of claim 1;
an impedance measuring unit electrically connected to the first and second lead wires, the unit configured to provide an output signal representative of a measured impedance of the conduction pathway;
an impedance controller configured to receive the output signal; and
an alarm responsive to the impedance controller configured to provide an indication to a user.

6. The electrophysiology catheter of claim 1 for use in or near a tubular region of a patient's heart, wherein:
the inflatable member is configured for placement in blood flow through the tubular region, and the inflatable member is configured to adopt the inflated configuration in or near the tubular region.

7. The catheter of claim 6, further comprising a shaft extending through the inflatable member, wherein the first electrode is mounted on the shaft distal of the inflatable member and the second electrode is mounted on the shaft proximal of the inflatable member.

8. The catheter of claim 6, wherein the inflatable member in the inflated configuration is configured to provide circumferential contact of an outer surface of the inflatable member with tissue wall of the tubular region to occlude the tubular region from blood flow.

9. The catheter of claim 8, wherein a plurality of ablation electrodes are affixed to the outer surface.

10. An electrophysiology system for detecting occlusion, comprising:
a catheter having:
an inflatable member configured for placement in blood flow from a pulmonary vein into a left atrium through an ostium, the inflatable member configured to adopt an inflated configuration in or near the ostium;
a first electrode distal of the inflatable member and configured for placement in the pulmonary vein, a second electrode proximal of the inflatable member and configured for placement in the left atrium, the first and second electrodes configured to define a conduction pathway between the pulmonary vein and the left atrium; and
a first lead wire connected to the first electrode and a second lead wire connected to the second electrode, the first and second lead wires configured to pass an electrical current between the first and second electrodes for measuring impedance of the conduction pathway;
an impedance measuring unit electrically connected to the first and second lead wires, the unit configured to provide an output signal representative of a measured impedance of the conduction pathway;
an impedance controller configured to receive the output signal; and
an alarm responsive to the impedance controller configured to provide an indication to a user.

11. The system of claim 5 or claim 10, wherein the impedance measuring unit includes a current/voltage source, a resistor, and a voltmeter.

12. The system of claim 5 or claim 10, wherein the impedance controller is configured to determine an occurrence of a measured impedance exceeding a threshold impedance value.

13. The system of claim 5 or claim 10, wherein the impedance controller is configured to determine a rate of change of measured impedance.

14. The system of claim 5 or claim 10, wherein the impedance controller is configured to determine an occurrence of a rate of change of measured impedance exceeding a threshold rate of change of impedance.

15. The system of claim 5 or claim 10, wherein the impedance controller includes an A/D converter, an impedance processor, a memory and a clock.
